# EUROPÄISCHE PATENTSCHRIFT

(11) **EP 0 808 815 B1**
(45) Veröffentlichungstag und Bekanntmachung des Hinweises auf die Patenterteilung: **16.08.2001**
(21) Anmeldenummer: 97105669.2
(22) Anmeldetag: 05.04.1997
(51) Int. Cl.: C07C 39/08, C07C 37/055, C07C 37/07, C07C 67/08

(54) **Verfahren zur Herstellung von Trimethylhydrochinon**
Method for the preparation of trimethylhydroquinone
Procédé pour la préparation de triméthylhydroquinone

(30) Priorität: 14.05.1996 DE 19619387; 11.07.1996 DE 19627977
(43) Veröffentlichungstag der Anmeldung: 26.11.1997
(73) Patentinhaber: Degussa AG, 40474 Düsseldorf (DE)
(72) Erfinder: Hübner, Frank, Dr., 64372 Ober-Ramstadt (DE); Krill, Steffen, Dr., 67346 Speyer (DE); Drapal, Bernd, 63755 Alzenau (DE); Schmitt, Hermann, 63517 Rodenbach (DE); Huthmacher, Klaus, Dr., 63571 Gelnhausen (DE); Tanner, Herbert, Dr., 63457 Hanau (DE)

(56) Entgegenhaltungen:
- DE-A- 2 149 159
- Y.A JOE ET AL: "Characteristic oxidative aromatization pattern of isophorone, 4-hydroxyisophorone and rearrangement of 4-oxoisophorone under a strong acidic condition" BULLETIN OF THE KOREAN CHEMICAL SOCIETY, Bd. 12, Nr. 3, 1991, Seiten 253-254, XP000672598

## Beschreibung

Die Erfindung betrifft ein verbessertes Verfahren zur Herstellung von 2,3,5-Trimethylhydrochinon durch Umlagerung von 4-Oxo-Isophoron (Keto-Isophoron, 3,5,5-Trimethylcyclohex-2-en-1,4-dion) zu einem Trimethylhydrochinondiester und dessen anschließende Verseifung.

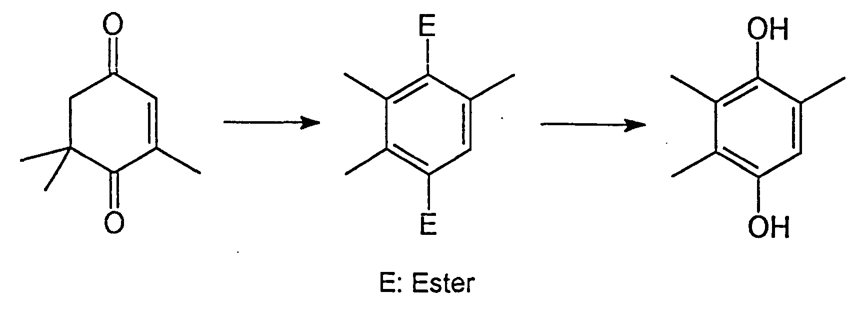

Trimethylhydrochinon wiederum ist ein wichtiges Ausgangsprodukt für die Herstellung von Vitamin E.

### Stand der Technik

Es ist bereits bekannt (DE 26 46 172 C2), Keto-Isophoron in der Gasphase mit einem Zeolithen in das Trimethylhydrochinon umzulagern. Die Ausbeuten bei dieser Reaktion sind allerdings nur gering (50 % bei 30 % Umsatz) und damit für ein ökonomisches Verfahren unbefriedigend. In einem weiteren Verfahren (Y.A. Joe, Y.M. Goo, Y.Y. Lee *Bull. Korean. Chem. Soc.* 1991, 12, 253) wird die Umlagerung in 5 %-iger Lösung in Acetanhydrid durch Zugabe von fünf Äquivalenten konzentrierter Schwefelsäure durchgeführt. Trimethylhydrochinonester werden dabei mit lediglich 31 % Ausbeute erhalten, so daß auch dieses Verfahren nicht wirtschaftlich ist.
Nach einer dritten Methode (DE-OS 2 149 159) kann Keto-Isophoron in Gegenwart einer Protonensäure in Acetanhydrid zu Trimethylhydrochinondiacetat umgesetzt werden, welches anschließend zu 2,3,5- Trimethylhydrochinon verseift wird. Nachteilig an diesem Verfahren ist die Verwendung
- großer Mengen an Acetanhydrid (5-10 mol/mol Ketoisophoron),
- großer Mengen der Katalysatorsäure (bis 150 mol%)
- sowie die mit maximal 66 % recht mäßige Ausbeute.

Es wurde nun ein Verfahren zur Herstellung von Trimethylhydrochinon (TMHQ) durch Umsetzung von Keto-Isophoron mit Acetanhydrid als Acylierungsmittel in Gegenwart katalytischer Mengen einer Protonensäure und anschließende Verseifung des zunächst gebildeten Trimethylhydrochinonesters gefunden,das dadurch gekennzeichnet ist, daß man als Protonensäure Trifluormethansulfonsäure, Chlorsulfonsäure, Polyphosphorsäure oder Oleum oder Mischungen dieser Säuren in einer Menge von 0,1 bis 50 Gew.-%,insbesondere 0,5 bis 25 Gew.-%, bezogen auf das Endion, einsetzt.
Bevorzugt verwendet man pro Mol Keto-Isophoron >2 bis 4 Mol, insbesondere 2,1 bis 3 mol des Acylierungsmittels. In einer bevorzugten Ausführungsform verseift man das entstandene TMHQ-Diacetat ohne Isolierung, gegebenenfalls nach dem Abdestillieren von nicht umgesetztem Acetanhydrid durch Zugabe von Wasser oder verdünnter Säure, insbesondere Schwefelsäure, und Erhitzen der Mischung zum Sieden.Das entstandene TMHQ wird anschließend abfiltriert.

Man kann aber auch das entstandene TMHQ-Diacetat nach der Zugabe von Wasser aus dem Reaktionsgemisch abtrennen, in verdünnter Säure, insbesondere Schwefelsäure in Gegenwart eines Phasenvermittlers hydrolysieren und das entstandene TMHQ abtrennen, insbesondere durch Filtrieren.

Als Phasenvermittler bei der Verseifung auch des isolierten Trimethylhydrochinondiesters können alle organischen Lösungsmittel Verwendung finden, die eine gewisse Mischbarkeit mit Wasser aufweisen. Besonders vorteilhaft können Essigsäure, n-Butanol und n-Butylacetat oder Mischungen der genannten Lösungsmittel eingesetzt werden.

### Durchführung

Zur Herstellung von 2,3,5 - Trimethylhydrochinon nach dem erfindungsgemäßen Verfahren werden in einem Eintopfverfahren beispielsweise 0,2 Mol Keto-Isophoron zu einer Mischung von >0,4-0,6 Mol Essigsäureanhydrid und 0,1-50 Gew.-%,insbesondere 0,5 - 25 Gew.-%, bezogen auf Keto-Isophoron, einer der genannten sehr starken Säuren innerhalb von 1 bis 3 h bei 0 - 60 ° C zugetropft und anschließend 1 bis 7 h auf ca. 25 - 70 ° C erwärmt. Danach werden durch Zugabe einer ausreichenden Menge Wasser Reste des Essigsäureanhydrids hydrolysiert. Zu der entstandenen Suspension gibt man gegebenenfalls Schwefelsäure, bevorzugt ca. 30 %ige, und erhitzt 1 bis 5 h zum Sieden. Anschließend wird ein Teil des Lösungsmittels abdestilliert und durch die gleiche Menge Wasser ersetzt, die Suspension auf Raumtemperatur abgekühlt und das ausgefallene Trimethylhydrochinon abtrennt.

Man kann ebenso nach der ersten Zugabe von Wasser den ausgefallenen Trimethylhydrochinondiester abtrennen und separat verseifen. Dazu wird der Trimethylhydrochinondiester z. B. in einer ausreichenden Menge einer verdünntenSäure, bevorzugt. 30 %iger Schwefelsäure, und einem Phasenvermittler, wie z. B. n-Butanol supendiert und anschließend 1 bis 7 h zum Sieden erhitzt. Danach wird Destillat abgenommen und anschließend dieselbe Menge an Wasser dem Sumpf zugesetzt. Das dann ausgefallene Trimethylhydrochinon wird abgetrennt und durch Nachwaschen gereinigt.

Die Erfindung wird im folgenden anhand von Ausführungsbeispielen weiter erläutert.

### Vorteile

Bei der erfindungsgemäßen Herstellung von 2,3,5-Trimethylhydrochinon ergeben sich erhebliche Vorteile gegenüber dem Stand der Technik:
- Die Ausbeuten sind nach dem erfindungsgemäßen Verfahren um bis zu 25 % höher als in der zitierten Literatur und liegen zwischen 85 und 90 %.
- Die benötigten Katalysatormengen liegen bei 0,1 bis 50 % gegenüber bis zu 150 % in der Literatur.
- Es werden lediglich >2 bis 4 Mol eines Carbonsäureanhydrids pro Mol Keto-Isophoron benötigt gegenüber 5 bis 10 Mol in der Literatur.
- Die Verseifung des isolierten Trimethylhydrochinondiesters mit wäßriger Säure gelingt bevorzugt in einfacher Weise in Gegenwart eines Phasenvermittlers.

### Beispiel 1:

Zu einer Lösung von 61 g (0,6 mol) Essigsäureanhydrid und 0,34 g (2,3 mmol) Trifluormethansulfonsäure wurden innerhalb 1,5 h 30,5 g (0,2 mol) Keto-Isophoron (98 %) bei 30° - 40° C zugetropft. Anschließend ließ man 3 h bei 40° C ausreagieren. Nach beendeter Reaktion wurde unter Kühlung mit 125 ml Wasser versetzt, das ausgefallene Trimethylhydrochinon-diacetat abgesaugt, nachgewaschen und 14 h bei 55° C im Vakuum getrocknet.
- Ausbeute:: 45,0 g (95 % d.Th.)
- GC:: 94,5 % TMHQ-Diacetat

### Beispiel 2:

In einer Mischung von 100 ml 30%iger Schwefelsäure und 15 ml n-Butanol wurden 43 g (0,18 mol) TMHQ-Diacetat unter Erwärmen gelöst und anschließend 4 h zum Sieden erhitzt. Danach wurde innerhalb von 45 min 80 ml Destillat abgenommen und mit 100 ml Wasser versetzt. Das ausgefallene Trimethylhydrochinon wurde bei 20° C abgesaugt, nachgewaschen und 14 h bei 55° C im Vakuum getrocknet.
- Ausbeute:: 26,2 g (95 % d. Th.)
- HPLC:: 98,5 %

### Beispiel 2a:

In einer weiteren Verseifung wurde anstelle des n-Butanols und der 30%igen Schwefelsäure eine Mischung von 50 ml des Destillates aus Versuch 2, 30 g Schwefelsäure und 50 ml Wasser verwendet.

### Beispiel 3:

Der Versuch des Beispiels 1 wurde wiederholt, wobei diesmal nach der Reaktion 30 g einer Mischung von Essigsäureanhydrid und Essigsäure abdestilliert wurde. Die übrige Aufarbeitung erfolgte analog.
- Ausbeute:: 45,5 g TMHQ-Diacetat (96 % d.Th.)

### Beispiel 4:

Der Versuch des Beispiels 2 wurde wiederholt, wobei diesmal vor der Filtration des Produktes die Mischung auf -10° C abgekühlt wurde.
- Ausbeute:: 26,9 g TMHQ (97 % d.Th.)
- HPLC:: 95 %

### Beispiel 5 - 7:

Der Versuch des Beispiels 1 wurde wiederholt, wobei anstelle der Trifluormethansulfonsäure diesmal andere Katalysatoren eingesetzt wurden.

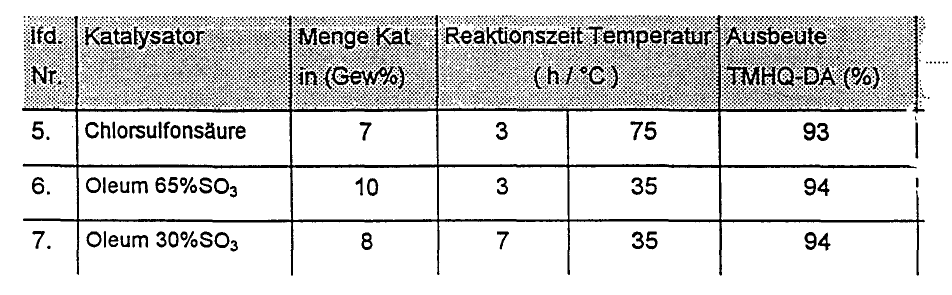

### Beispiel 8: (Direktvariante)

Zu einer Mischung von 61 g (0,6 mol) Essigsäureanhydrid und 3 g Oleum (65% SO₃) wurden 30,5 g (0,2 mol) Keto-Isophoron innerhalb von 1,5 h bei 10 - 25° C zugetropft und anschließend 4 h auf 40° C erwärmt. Danach wurde durch Zugabe von 90 ml Wasser hydrolysiert. Zur entstandenen Suspension gab man 47 g Schwefelsäure und erhitzte 3 h zum Sieden.
Die weitere Aufarbeitung erfolgte analog Beispiel 2.
- Ausbeute:: 27 g TMHQ (89 % d.Th.)
- HPLC:: 98,5 %

## Patentansprüche

1. Verfahren zur Herstellung von Trimethylhydrochinon (TMHQ)durch Umsetzung von Keto-Isophoron mit Acetanhydrid als Acylierungsmittel in Gegenwart katalytischer Mengen einer Protonensäure und anschließende Verseifung des zunächst gebildeten Trimethylhydrochinonesters,
dadurch gekennzeichnet, daß man als Protonensäure Trifluormethansulfonsäure, Chlorsulfonsäure, Polyphosphorsäure oder Oleum oder Mischungen dieser Säuren in einer Menge von 0,1 bis 50 Gew.-%, bezogen auf das Endion, einsetzt.

2. Verfahren gemäß den Anspruchen 1,
durch gekennzeichnet, daß man pro Mol Keto-Isophoron >2 bis 4 Mol des Acylierungsmittels einsetzt.

3. Verfahren gemäß Anspruch 1,
dadurch gekennzeichnet, daß man das entstandene TMHQ-Diacetat ohne Isolierung, gegebenenfalls nach dem Abdestillieren von nicht umgesetztem Acetanhydrid, durch Zugabe von Wasser und/oder verdünnter Säure verseift und das entstandene TMHQ abtrennt.

4. Verfahren gemäß Anspruch 1,
dadurch gekennzeichnet, daß man das entstandene TMHQ-Diacetat nach der Zugabe von Wasser aus dem Reaktionsgemisch abtrennt, unter Verwendung verdünnter Säure in Gegenwart eines Phasenvermittlers verseift und das entstandene TMHQ abtrennt.

5. Verfahren gemäß Anspruch 4,
dadurch gekennzeichnet, daß man als Phasenvermittler Essigsäure, n-Butanol oder n-Butylacetat oder deren Gemische einsetzt.

## Claims

1. Method for the preparation of trimethylhydroquinone (TMHQ) by reaction of ketoisophorone with acetic anhydride as acylating agent in the presence of catalytic quantities of a protonic acid and subsequent saponification of the initially formed trimethylhydroquinone ester, characterised in that trifluoromethanesulfonic acid, chlorosulfonic acid, polyphosphoric acid or oleum or mixtures of these acids in a quantity of 0.1 to 50 wt.%, based on the end ion, are used as protonic acid.

2. Method according to claim 1,
characterised in that >2 to 4 mol of the acylating agent is used per mol ketoisophorone.

3. Method according to claim 1,
characterised in that the TMHQ diacetate formed, without isolation, optionally after the removal of unreacted acetic anhydride by distillation, is saponified by addition of water and/or dilute acid and the TMHQ formed is separated off.

4. Method according to claim 1,
characterised in that the TMHQ diacetate formed is separated from the reaction mixture after the addition of water, is saponified using dilute acid in the presence of a phase promoter and the TMHQ formed is separated off.

5. Method according to claim 4,
characterised in that acetic acid, n-butanol or n-butyl acetate or mixtures thereof are used as phase promoter.

## Revendications

1. Procédé pour la préparation de triméthylhydroquinone (TMHQ) par réaction de ceto-isophorone avec de l'anhydride acétique comme agent d'acylation en présence de quantités catalytiques d'un acide protonique et saponification consécutive de l'ester de la triméthylhydroquinone formé initialement, caractérisé en ce qu'on utilise comme acide protonique de l'acide trifluorométhanesulfonique, de l'acide chlorosulfonique, de l'acide polyphosphorique ou de l'oléum ou des mélanges de ces acides en une quantité de 0,1 à 50 % en poids, par rapport à la céto-isophorone.

2. Procédé selon la revendication 1, caractérisé en ce qu'on utilise >2 à 4 moles d'agent d'acétylation par mole de céto-isophorone.

3. Procédé selon la revendication 1, caractérisé en ce saponifie le diacétate de TMHQ formé sans isolation, le cas échéant après élimination par distillation de l'anhydride acétique non transformé, par addition d'eau et/ou d'acide dilué et qu'on sépare la TMHQ formée.

4. Procédé selon la revendication 1, caractérisé en ce qu'on sépare le diacétate de TMHQ formé après l'addition d'eau du mélange réactionnel, qu'on saponifie en utilisant de l'acide dilué en présence d'un promoteur des phases et qu'on sépare la TMHQ formée.

5. Procédé selon la revendication 4, caractérisé en ce qu'on utilise comme promoteur de phases de l'acide acétique, du n-butanol ou de l'acétate de n-butyle ou leurs mélanges.
